# NEUE EUROPÄISCHE PATENTSCHRIFT

(11) **EP 1 920 044 B2**
(45) Veröffentlichungstag und Bekanntmachung des Hinweises auf die Entscheidung über den Einspruch: **17.06.2020**
(45) Hinweis auf die Patenterteilung: 01.07.2009
(21) Anmeldenummer: 06776498.5
(22) Anmeldetag: 29.07.2006
(51) Int. Cl.: C12M 1/00

(54) **SYSTEM AUS MEHREREN INKUBATOREN**
SYSTEM COMPRISED OF A NUMBER OF INCUBATORS
SYSTEME DE PLUSIEURS INCUBATEURS

(30) Priorität: 04.08.2005 DE 102005036763
(43) Veröffentlichungstag der Anmeldung: 14.05.2008
(73) Patentinhaber: Inheco Industrial Heating and Cooling GmbH, 82152 Martinsried (DE)
(72) Erfinder: GEORGE, Christian, 82515 Wolfratshausen (DE); MOMBOISSE, Michel, 81669 München (DE); BURDACK, Torsten, 80687 München (DE); PUKLOWSKY, Ralf, 85635 Höhenkirchen-Siegertsbrunn (DE); MARINO, Giuseppe, 80339 München (DE); TENZLER, Guenter, 80638 München (DE)
(74) Vertreter: Grättinger Möhring von Poschinger Patentanwälte Partnerschaft mbB
(86) Internationale Anmeldenummer: PCT/EP2006/007533
(87) Internationale Veröffentlichungsnummer: WO 2007/014727

(56) Entgegenhaltungen:
- WO-A-03/061830
- DE-B4- 10 134 885
- US-A- 4 759 008
- US-A- 5 123 477
- US-A1- 2004 152 188
- US-B1- 6 518 059

## Beschreibung

Die vorliegende Erfindung betrifft ein System aus mehreren Inkubatoren, die jeweils ein Gehäuse, eine innerhalb des Gehäuses angeordnete Probenkammer zur Aufnahme eines Probenbehältnisses, eine Temperiereinheit und eine die Temperiereinheit steuernde Steuerelektronik umfassen, wobei die Probenkammern der Inkubatoren mittels der Temperiereinheiten beheiz- und/oder kühlbar sind und die Gehäuse der Inkubatoren jeweils eine verschließbare Zugangsöffnung zum Be- und Entladen der Probenkammer aufweisen, wobei die Gehäuse der Inkubatoren ein vertikales Stapeln der Inkubatoren gestatten.

Derartige Inkubatoren finden vorzugsweise Anwendung für Proben aus den Bereichen der Biologie, Biotechnologie, Pharmazie, Diagnostik oder der Chemie, die in der Probenkammer des Inkubators für einen gewissen Zeitraum mit definierten Umgebungsbedingungen beaufschlagt werden sollen. Hierzu werden die Proben in einem Probenbehältnis in die Probenkammer des Inkubators gebracht. Im Falle von flüssigen Proben handelt es sich bei den Probenbehältnissen um Flüssigkeitsbehälter mit jeweils einem oder mehreren Teilbehältnissen, wie z.B. um sogenannte "microplates" oder "deep-well-microplates".

Ein System aus mehreren Inkubatoren zur Aufnahme von "microplates" mit den gattungsbildenden Merkmalen der vorliegenden Erfindung ist beispielsweise aus der US 6518059 B1 bekannt. Dabei sind mehrere - in ihrer Funktionalität identische - Inkubatoren übereinander stapelbar. Gemäß zweier alternativer Ausführungen ist ferner vorgesehen, daß die Temperatur in den Probenkammern der Inkubatoren entweder an jedem Inkubator separat über dessen eigene Temperaturkontrolleinheit regelbar ist oder über die Temperaturkontrolleinheit eines sogenannten Masterinkubators steuerbar ist. In letzterem Fall können die Inkubatoren nur bei im wesentlichen gleicher Temperatur in der Probenkammer betrieben werden.

Ausgehend von dem vorbekannten Stand der Technik ist es die Aufgabe der vorliegenden Erfindung, ein möglichst universell einsetzbares modulares System aus mehreren Inkubatoren der gattungsgemäßen Art zur Verfügung zu stellen, das möglichst einfach erweiterbar bzw. rückbaubar ist, eine möglichst hohe Funktionalität - auch in Zusammenhang mit weiteren Automatisierungsgeräten - bereitstellt, die Möglichkeit bietet, verschiedene Proben gleichzeitig mit unterschiedlichen Umgebungsbedingungen zu beaufschlagen und schließlich erlaubt, Inkubatoren verschiedener Bauart flexibel zu einem System zu verbinden.

Diese Aufgabe wird durch ein gattungsgemäßes System mit den weiteren Merkmalen des Anspruchs 1 gelöst, indem nämlich die Inkubatoren jeweils ein mit ihrer Steuerelektronik zusammenwirkendes Bussystem aufweisen, wobei die Bussysteme der Inkubatoren über entsprechende Anschlußelemente untereinander verbunden sind, und das System eine zentrale Steuereinheit umfaßt, die mit dem Bussystem eines der Inkubatoren des Systems (Masterinkubator) verbunden ist, wobei über die Bussystemarchitektur ein individuelles Ansteuern der Steuerelektronik sämtlicher Inkubatoren des Systems durch die zentrale Steuereinheit möglich ist, so daß Proben in verschiedenen Probenkammern gleichzeitig einer unterschiedlichen Temperatur ausgesetzt werden können. Erfindungsgemäß ist ferner vorgesehen, daß wenigstens ein Inkubator des Systems sich von den anderen Inkubatoren des Systems hinsichtlich seiner Bauart und Funktionalität unterscheidet, indem er neben einer Temperiereinheit wenigstens ein weiteres Mittel zur Beeinflussung weiterer Umgebungsbedingungen für die in der Probenkammer befindlichen Proben aufweist.

Das erfindungsgemäße System zeichnet sich somit dadurch aus, daß lediglich ein Inkubator - der sogenannte Masterinkubator - mit einer zentralen Steuereinheit verbunden werden muß. In die Inkubatoren eingebaute und untereinander verbundene Bussysteme erlauben ein separates und individuelles Ansteuern der Steuerelektronik sämtlicher Inkubatoren des Systems mit Hilfe der zentralen Steuereinheit. Damit ist es - bei gleichzeitiger Modularität - möglich, Proben in verschiedenen Probenkammern gleichzeitig einer unterschiedlichen Temperatur auszusetzen. Das erfindungsgemäße System ist äußerst einfach erweiterbar, da ein neu zum System hinzukommender Inkubator nicht direkt mit der zentralen Steuereinheit verbunden werden muß, um dessen individuelle Ansteuerbarkeit zu gewährleisten. Vielmehr ist nur das mit seiner Steuerelektronik zusammenwirkende Bussystem an das Bussystem eines der bereits zuvor im System vorhandenen Inkubatoren anzuschließen, um eine Ansteuerung der Steuerelektronik des neu hinzugekommenen Inkubators über die Bussystemarchitektur mit der zentralen Steuereinheit zu ermöglichen.

Ferner eröffnet das erfindungsgemäße System dem Anwender die im bisherigen Stand der Technik nicht gegebene Möglichkeit, auch Inkubatoren verschiedener Bauart und Funktionalität in das System zu integrieren, sofern sie die in Anspruch 1 näher definierten Merkmale und insbesondere ein mit ihrer Steuerelektronik zusammenwirkendes und untereinander kompatibles Bussystem aufweisen.

Beispielsweise können somit Inkubatoren, deren Probenkammern mittels einer Temperiereinheit nur beheizbar sind, auf einfache Art und Weise um einen Inkubator ergänzt werden, dessen Probenkammer nur kühlbar ist.

Ersichtlich können sich die Inkubatoren auch in anderen zusätzlichen Funktionalitäten unterscheiden, auf die weiter unten eingegangen werden wird.

Bei der konkreten Ausgestaltung der Bussysteme der Inkubatoren und der Anbindung der Bussysteme an die jeweilige Steuerelektronik bedient sich der Fachmann des für Bussysteme maßgeblichen Stands der Technik, aus dem Bussysteme in unterschiedlichsten technischen Ausführungen bekannt sind.

Vorteilhaft werden dabei neu dem System hinzugefügte Inkubatoren automatisch von der zentralen Steuereinheit erkannt und diesen ggfs. gleich eine Adresse im Bussystem zugeordnet, über die sie ansteuerbar sein sollen. Ferner weist das Bussystem jedes Inkubators vorteilhaft Mittel zur Identifizierung seines Typs bzw. seiner Funktionalität auf. Mit den vorstehenden Merkmalen stellt das System eine sogenannte "Plug-and-Play"-Funktionalität bereit, die ein besonders einfaches Inbetriebnehemen eines hinzugefügten Inkubators erlaubt. Weiterhin kann vorgesehen sein, sofern sich die räumliche Lage eines neu hinzugefügten Inkubators zwingend anhand seines Anschlusses an das Bussystem ergibt (siehe unten), daß dem Inkubator sogleich entsprechende Raumkoordinaten zugeordnet werden, was das Zusammenspiel mit einem automatisierten Robotersystem, beispielsweise zum Be- und Entladen der Probenkammern der Inkubatoren, erheblich erleichtert.

Gemäß einer ersten vorteilhaften Ausgestaltung des erfindungsgemäßen Systems kann vorgesehen sein, daß jeweils zwei benachbarte Inkubatoren des Systems über zwei Anschlußelemente in Form von zueinander korrespondierenden Steckelementen verbindbar sind, wobei zumindest bei einem Teil der Inkubatoren die Steckelemente derart einander gegenüberliegend an der Oberseite und der Unterseite ihres Gehäuses angeordnet sind, daß sie mit wiederum korrespondierenden Steckelementen des darunter liegenden und/oder darüber gestapelten Inkubators eine - direkte - Steckverbindung bilden.
Ersichtlich sind hierbei bevorzugt alle, jeweils einen oberen und unteren "Nachbarinkubator" aufweisenden Inkubatoren im Stapel mit zwei derartigen Anschlußelementen ausgestattet. Die beiden den Stapel unten bzw. oben begrenzenden Inkubatoren benötigen ein entsprechendes Anschlußelement eigentlich nur auf ihrer Ober- bzw. Unterseite. Es erweist sich für die Erweiterbarkeit des Systems jedoch als vorteilhaft, wenn auch der oberste Inkubator des Stapels mit zwei entsprechenden Anschlußelementen in Form einander korrespondierender Steckelemente ausgestattet ist, da dann noch mindestens ein weiterer Inkubator - mit zumindest einem entsprechenden Steckelement auf seiner Unterseite - durch einfaches Darüberstapeln nachrüstbar ist.

Bei der vorstehend geschilderten Ausgestaltung des Systems müssen keine zusätzlichen Verbindungskabel bereitgestellt und angeschlossen werden. Die Verbindung der Bussysteme erfolgt vielmehr bereits direkt beim Stapeln eines neu hinzukommenden Inkubators auf den bereits vorhandenen Stapel. Dies ermöglicht es insbesondere auch, einem hinzugekommenen Inkubator automatisch die seiner räumlichen Lage entsprechenden Raumkoordinaten zuzuordnen, da er zwingend direkt über dem obersten Inkubator eines Stapels angeordnet ist und sich seine Position relativ zum Masterinkubator aus den vorbekannten geometrischen Abmessungen des Inkubators - ggfs. abhängig von seiner über das Bussystem identifizierbaren Bauart - ableiten läßt.

Erkennbar ist es bei der vorstehend beschriebenen Ausgestaltung und Anordnung der Anschlußelemente der Inkubatoren - im Sinne eines möglichst einfachen Rückbaus des Systems - von besonderem Vorteil, wenn der mit der zentralen Steuereinheit verbundene Masterinkubator den untersten Inkubator in dem erfindungsgemäß gestapelten System aus mehreren Inkubatoren bildet. Hierzu ist sein Bussystem vorteilhaft, falls er ein Steckelement an der Unterseite seines Gehäuses aufweist, über dieses oder ggfs. auch über ein anderes Anschlußelement mit der zentralen Steuereinheit verbunden.

In einer weiteren vorteilhaften Ausgestaltung des Systems, weist zumindest ein Teil der Inkubatoren eine in Linearführungen geführte Schublade zur Be- und Entladung der Probenkammer auf, die mittels eines Motors durch die - vorzugsweise frontseitig angeordnete - Zugangsöffnung aus dem Gehäuse des Inkubators heraus- und in diesen wieder hereinfahrbar ist.

Dadurch können die Inkubatoren des Systems automatisiert be- und entladen werden. Vorteilhaft weist der Inkubator somit auch eine - bevorzugt über das Bussystem steuerbare - Steuerelektronik für die Antriebseinheit der Schublade auf, bei der es sich wiederum bevorzugt um die bereits zur Steuerung der Temperiereinheit vorhandene Steuerelektronik handeln kann, die somit eine weitere Funktion übernimmt. Die Proben bzw. Probenbehältnisse können der vollständig ausgefahrenen Schublade dann von einem - vorzugsweise über die zentrale Steuereinheit gesteuerten - Robotersystem zugeführt und dort abgesetzt werden. Bei dem obersten Inkubator eines Stapels kann ggfs. auf eine solche Schublade verzichtet werden, da dieser auch durch eine an seiner Oberseite angeordnete Zugangsöffnung automatisiert be- und entladen werden könnte.

Die Schublade der Inkubatoren muß zum Tragen der Probe bzw. des Probenbehältnisses geeignet sein und kann zugleich den Boden der Probenkammer bilden. Hierzu kann sie beispielsweise vollflächig ausgestaltet sein. Dies ist indessen nicht zwingend; es kann sich bei der Schublade beispielsweise auch um eine rahmenförmige Aufnahme handeln, die die Probenbehältnisse nur in deren Randbereich trägt.

Insbesondere erweist es sich als vorteilhaft, wenn die Schublade in beiden.Linearführungen vertikal fest gelagert, hingegen horizontal quer zu ihrer Bewegungsrichtung in einer der Linearführungen fest und in der anderen Linearführung lose gelagert ist. Dies gestattet eine gewisse Wärmedehnung der Schublade und verhindert damit ein Verklemmen im Dehnfall.

In einer abermals bevorzugten Ausgestaltung des Systems liegt die Schublade in ihrer vollständig ausgefahrenen Endposition an mindestens einem oder mehreren Anschlägen des betreffenden Inkubators an. Dies ist mit entsprechend präzise tolerierten Anschlägen in Zusammenhang mit einem automatisierbaren Robotersystem zum Handling der Probenbehältnisse von besonderem Vorteil, da durch den mindestens einen Anschlag die Lage der vollständig ausgefahrenen Schublade auch nach vielen Öffnungs- und Schließzyklen noch sehr gut reproduzierbar ist, womit ein Fehlgreifen oder ein Fehlabsetzen der Probenbehältnisse durch den Roboter vermieden wird.

Gemäß einer vorteilhaften Weiterbildung des Systems wird die frontseitige Zugangsöffnung zur Probenkammer des Inkubators durch eine stirnseitig an der Schublade befestigte Klappe verschlossen. Im Gegensatz zu einer am Gehäuse des Inkubators angebrachten Klappe muß somit kein separater Öffnungsmechanismus für diese vorgesehen sein. Auch kann die Schließbewegung der Schublade in ein aktives Schließen der Klappe umgesetzt werde.

Eine wiederum bevorzugte Weiterbildung des Systems sieht vor, daß die Klappe bei hereingefahrener Schublade eine im wesentlichen vertikale, die Zugangsöffnung verschließende Stellung einnimmt und an der Schublade um eine horizontale Achse derart schwenkbar befestigt ist, daß sie bei geöffneter Schublade in eine im wesentlichen horizontale Stellung wegklappt. Hierzu kann die Klappe z.B. über eine Federeinrichtung vorgespannt sein. Damit steht sie in ihrer im wesentlichen horizontalen Stellung einem etwa für das Handling der Probenbehältnisse eingesetzten Greifarm eines Robotersystems nicht im Wege.

Die Schwenkachse zwischen Schublade und Klappe ist klappenseitig vorteilhaft entfernt von deren - in vertikaler Stellung gesehenen - unterem und oberem Rand angeordnet. Durch diese vom Rand entfernte und damit eher mittige Anordnung kann beim Schließen der Zugangsöffnung des Gehäuses beim Hereinfahren der Schublade eine über die Klappe im wesentlichen gleichverteilte Schließkraft aufgebracht werden. Ferner verringert sich im Unterschied zu einer randseitigen Schwenkachse der beim Wegklappen erforderliche Raum.

Dabei muß die Klappe beim Schließen der Schublade durch geeignete Mittel wieder in ihre aufrechte bzw. vertikale Stellung gebracht werden, um die Zugangsöffnung zur Probenkammer ordnungsgemäß zu verschließen. Dies kann vorteilhaft über eine entsprechende Kurvenführung am Gehäuse geschehen, an der ein Teil der Klappe beim Schließen der Schublade anschlägt und derart umgelenkt wird, daß sich die Klappe wieder aufrichtet.

Durch die vorstehend erläuterte Ausgestaltung des Systems kann dieses mit Roboter- bzw. Greifsystemen unterschiedlicher Bauart zusammenarbeiten. Insbesondere für microplates oder andere standardisierte, längliche Probenbehältnisse sind bereits verschiedene Robotersysteme bekannt, die die Probenbehältnisse zu deren Transport an ihren Längs- bzw. Querseiten im sogenannten Portrait- bzw. Landscape-Modus greifen. Die linear geführte Schublade mit frontseitig angebrachter Klappe erlaubt ein Greifen bzw. Absetzten der Labware im Portrait-Modus. Durch ein Wegklappen der Klappe in eine im wesentlichen horizontale Lage wird zudem ein Greifen von Probenbehältnissen im Landscape-Modus erlaubt.

Vorteilhaft weisen die Inkubatoren des erfindungsgemäßen Systems Mittel zum Aufbringen einer gewissen Schließkraft auf die geschlossene Klappe auf. Hierzu kann z.B. ein als Elektromotor ausgebildeter Antriebsmotor für die Schublade in geschlossenem Zustand derart mit einem konstanten Motorstrom versorgt werden, daß die Klappe durch die Motorkraft verschlossen gehalten wird. Zum anderen können in bevorzugter Weise auch - ggfs. schaltbare - Magnetelemente am Inkubator und/oder an der Klappe vorgesehen sein, um ein ausreichend dichtes Schließen der Klappe zu gewährleisten.

In einer weiteren vorteilhaften Ausgestaltung des Systems umfaßt die Temperiereinheit mindestens eines Inkubators eine am Boden der Probenkammer angeordnete Heizfolie, mit der die Probenkammer und die darin befindliche Probe beheizbar sind.

In einer vorteilhaften Weiterbildung umfaßt die Temperiereinheit entweder eine Zwei-Zonen-Heizfolie oder mindestens zwei Heizfolien, mit denen mindestens zwei Zonen separat beheizbar sind. Dabei deckt vorteilhaft eine der Zonen einen umlaufenden Randbereich des Bodens der Probenkammer ab, während die zweite Zone den innen liegenden Kernbereich des Bodens der Probenkammer abdeckt. Beim Aufheizen der Probenkammer auf die gewünschte Betriebstemperatur steht vorteilhafte die gesamte Heizleistung aller Heizzonen zur Verfügung, während zur Beibehaltung einer bereits erreichten Betriebstemperatur vorteilhaft nur eine außen im Randbereich der Probenkammer liegende Heizzone in einem auf die Solltemperatur geregelten Betrieb bleibt. Damit kann eine besonders homogene Temperaturverteilung innerhalb der Probenkammer - und damit auch innerhalb der Probe selbst - erreicht werden, da die größten Wärmeverluste in den Randbereichen der Probenkammer auftreten. Ferner kann die Heizleistungsdichte im Randbereich bevorzugt noch zusätzlich derart angepaßt sein, daß verschiedene Wärmeübergangsbedingungen an den Ecken der Probenkammer oder an den Längs- und Querseiten ausgeglichen werden.

Die Temperiereinheit mindestens eines Inkubators des erfindungsgemäßen Systems kann vorteilhaft auch ein Kühlaggregat oder ein kombiniertes Kühl-/Heizaggregat umfassen. Damit sind vorteilhaft nur der Boden und/oder die Seitenwände der Probenkammer direkt kühlbar, während die Decke der Probenkammer nicht direkt gekühlt wird. Ferner kommen hierzu vorteilhaft auf dem thermoelektrischen Effekt basierende Peltier-Module zum Einsatz, die - je nach Vorzeichen der Betriebsspannung - sowohl ein Kühlen als auch ein Heizen erlauben. Durch Kühlen der Seitenwände wird - analog zum Beheizen eines äußeren Randbereichs des Bodens der Probenkammer - eine weitgehend gleichmäßige Temperaturverteilung realisiert. Ein im Vergleich zu einer Kernzone stärkeres Kühlen der Randbereiche des Bodens der Probenkammer erweist sich mit Peltier-Modulen - anders als bei einer Heizfolie - als sehr aufwendig. Dadurch, daß die Decke der Probenkammer ausdrücklich nicht direkt gekühlt wird, kann eine Kondensatbildung an der Decke und ein damit verbundenes Tropfen von Kondensat auf die sich im Probenbehältnis befindliche(n) Probe(n) wirksam vermieden werden.

Im Interesse einer möglichst geringen Bauhöhe der Inkubatoren des Systems kann ferner vorgesehen sein, daß Boden und/oder Seitenwände der Probenkammer von dem eigentlichen Kühlaggregat bzw. dem kombinierten Heiz- /Kühlaggregat räumlich getrennt und mit diesem über sogenannte Heatpipes verbunden sind. Die Temperiereinheit umfaßt dann vorteilhaft eine Kühl- bzw. Heizaggregat/Lüfter-Einheit sowie einen Kühl- bzw. Heizflansch, welcher einerseits mit dem Kühl- bzw. Heizaggregat in Verbindung steht und andererseits die Heatpipes kontaktiert. Damit kann das Kühl- bzw. Heizaggregat auch seitlich oder hinter der Probenkammer im Inkubator positioniert werden. Vorteilhaft befindet sich der Kühl- bzw. Heizflansch auf gleicher Höhe mit einem zu kühlenden bzw. zu heizenden Boden. Ein im wesentlichen horizontaler Betrieb der Heatpipe(s) resultiert nämlich in einer besonders guten Heiz- bzw. Kühlleistung.

In einer weiteren vorteilhaften Ausgestaltung des Systems umfaßt die Isolation mindestens eines der beheiz- und/oder kühlbaren Inkubatoren eine Sandwichstruktur aus mindestens zwei verschiedenen Isolationssystemen, von denen die äußere als Vakuumisolation ausgeführt ist. Das innere Isolationssystem kann vorteilhaft eine Schicht aus massivem Kunststoff oder eine Schicht aus geschlossen-zelligem Kautschuk sein. Nochmals bevorzugt, insbesondere im Hinblick auf eine möglichst geringe Dicke der Isolation und somit eine besonders geringe Bauhöhe der Inkubatoren, sind es insgesamt drei Isolationsschichten aus - von innen nach außen - massivem Kunststoff, geschlossen-zelligem Kautschuk und einer Vakuumisolation. Eine geringe Bauhöhe der Inkubatoren wirkt sich auch auf das System als solches äußerst positiv aus, da somit bei gleicher Zahl von Inkubatoren eine geringere Stapelhöhe erreicht wird, was wiederum die Stabilität eines solchen Stapels erhöht. Eine gute Isolation ist bei dem vorliegenden System auch deshalb besonders wichtig, da die in einem Stapel benachbarten Inkubatoren ggfs. bei unterschiedlichen Temperaturen betreibbar sein sollen. Durch die vorstehend geschilderte Isolation kann somit eine gegenseitige Beeinflussung von benachbarten Inkubatoren verringert werden.

Geschlossen-zelliger Kautschuk erzielt seine gute Isolationswirkung durch innerhalb des Kautschuks eingeschlossene Luftzellen. Die Vakuum-Isolation umfaßt bevorzugt einen auf einen Druck von kleiner 5 mbar evakuierten Dämmkern aus mikroporöser Kieselsäure, der mit einer Umhüllung aus metallisch bedampfter Mehrschichtfolie vakuumdicht umgeben ist; diese hat sich bei einer Dicke von nur 4mm in der Praxis - ohne weitere Isolationsmittel - bis hin zu Temperaturen von ca. 60° C in der Probenkammer als ausreichend erwiesen. Bei dem erfindungsgemäßen System kann das System jedoch vorteilhaft auch mindestens einen Inkubator umfassen, dessen Temperiereinheit eine Temperatur in der Probenkammer von bis zu 80° C oder sogar noch darüber (z.B. 100° C oder 105° C) erreicht. Bei der vorstehend beschriebenen Zwei- oder Drei-Schicht-Isolation sind die inneren Schichten aus Kunststoff und/oder Kautschuk vorteilhaft derart dimensioniert, daß sie im Regelbetrieb eine Innentemperatur von 80° C oder mehr in der Probenkammer auf 60° an der Außenschicht der zweiten Isolationsschicht reduzieren, so daß die Vakuum-Isolation die Restwärme ausreichend zu isolieren vermag.

Bei einer abermals vorteilhaften Ausgestaltung des erfindungsgemäßen Systems, bei dem die Inkubatoren eine Schublade zum Be- und Entladen der Probenkammer aufweisen, umfaßt die Schublade eine mittels einer Kulissenführung geführte Aufnahme für ein Probenbehältnis. Die Kulissenführung bewirkt dabei eine vertikale Absenkung der Aufnahme während des letzten Abschnittes des horizontalen Hereinfahrens der Schublade in das Gehäuse, wobei das Probenbehältnis auf einer Zwischenplatte oder dem Boden der Probenkammer abgestellt wird. Ein Abstellen der Probenbehältnisse auf dem Boden der Probenkammer erweist sich insbesondere dann als sinnvoll, wenn der Boden beispielsweise über Heatpipes mit einer Temperiereinheit statisch verbunden ist und somit nicht selbst als Teil der Schublade zum Be- und Entladen der Probenkammer genutzt werden kann. Damit ergibt sich der Vorteil, daß das Probenbehältnis direkt auf dem kühlbaren und/oder beheizbaren Boden der Probenkammer abgestellt wird, was einen besseren Wärmeübertrag auf die Probe zur Folge hat.

Die Probe bzw. das Probenbehältnis können jedoch auch auf einer - von dem Boden der Probenkammer verschiedenen - Zwischenplatte abgesetzt werden. Diese kann dabei - insbesondere bei einer nicht ebenen Unterseite der verwendeten Probenbehältnisse - als Adapterplatte zur Sicherstellung eines möglichst guten Übertrags der Heiz- und/oder Kühlleistung einer beheiz- und/oder kühlbaren Bodenplatte ausgestaltet sein, indem sie in ihrer Geometrie auf ihrer Unterseite dem Boden der Probenkammer und auf ihrer Oberseite der Geometrie des in die Probenkammer einzubringenden Probenbehältnisses angepaßt ist. Vorteilhaft ist die Zwischenplatte in einem solchen Fall aus Aluminium oder einem in seiner Wärmeleitfähigkeit vergleichbaren oder besseren Werkstoff hergestellt.

Alternativ hierzu kann jedoch auch vorgesehen sein, daß es sich bei der Zwischenplatte um die Oberseite eines in den Inkubator integrierten Shakertisches handelt, mit dem die Probe geschüttelt werden kann. Hierzu ist die Zwischenplatte vorteilhaft innerhalb der horizontalen Ebene bewegbar angeordnet und mittels eines geeigneten Antriebssystems zu Schwingungen innerhalb der horizontalen Ebene anregbar. Diese Shakereinheit wird bevorzugt - wie im übrigen vorteilhaft sämtliche elektronischen Komponenten der Inkubatoren des erfindungsgemäßen Systems - über die Steuerelektronik des Inkubators gesteuert und ist somit ebenfalls über das Bussystem von der zentralen Steuereinheit ansteuerbar.

Neben den bisher beschriebenen Mitteln zur Beeinflussung von Umgebungsbedingungen für die in der Probenkammer befindlichen Proben (z.B. Temperiereinheit für die Temperatur, optionaler Shakertisch zur Einleitung einer horizontalen Schüttelbewegung) kann mindestens ein Inkubator des erfindungsgemäßen Systems auch mindestens ein weiteres Mittel zur Beeinflussung weiterer Umgebungsbedingungen aufweisen. Darunter fallen insbesondere eine entsprechende Lichtquelle zur Beleuchtung der Proben mit UV- oder infrarotem Licht, eine Antenne zur Bestrahlung von Proben mit elektromagnetischen Feldern in verschiedenen Frequenzbereichen, eine ggfs. elektronisch schaltbare Gaseintritts- und Gasaustrittsöffnung, um die Proben z.B. mit Stickstoff (N2) oder einem anderen Gas zu begasen, Mittel zur Erhöhung oder Erniedrigung der Luftfeuchtigkeit in der Probenkammer, etc. Bei den vorliegenden Mitteln zur Beeinflussung der Umgebungsbedingungen in der Probenkammer kann in einer vorteilhaften Ausgestaltung insbesondere vorgesehen sein, daß diese auch über die Steuerelektronik und damit über die zentrale Steuereinheit ansteuerbar sind. Insbesondere in Zusammenhang mit dem erfindungsgemäß vorgesehenen Bussystem, das vorteilhaft automatisch die Bauart und Funktionalität verschiedner an das System anzuschließender Inkubatoren via "Plug-and-Play" erkennt, erweist sich damit das erfindungsgemäße System als äußerst anwenderfreundlich.

Ersichtlich können die verschiedenen vorstehend näher erläuterten vorteilhaften Ausgestaltungen des Systems bzw. der Inkubatoren beliebig kombiniert werden.

Das erfindungsgemäße System umfasst insbesondere einen Inkubator (Masterinkubator), der ein Gehäuse, eine innerhalb des Gehäuses angeordnete Probenkammer zur Aufnahme eines Probenbehältnisses, eine Temperiereinheit und eine die Temperiereinheit steuernde Steuerelektronik umfaßt, wobei die Probenkammer mittels der Temperiereinheit beheiz- und/oder kühlbar ist und das Gehäuse eine verschließbare Zugangsöffnung zur Beschickung der Probenkammer aufweist, wobei das Gehäuse geeignet ist, mindestens einen weiteren, vertikal auf diesem gestapelten Inkubator zu tragen. Er ist ferner dadurch gekennzeichnet, daß er ein mit seiner Steuerelektronik zusammenwirkendes Bussystem und zwei mit dem Bussystem verbundene Anschlußelemente aufweist, wobei sein Bussystem über das erste Anschlußelement mit einer zentralen Steuereinheit und über das zweite Anschlußelement mit einem Bussystem eines weiteren Inkubators (Slaveinkubator) verbindbar ist, so daß über die Bussystemarchitektur auch ein individuelles Ansteuern der Steuerelektronik des Inkubators und des weiteren Inkubators durch die zentrale Steuereinheit möglich ist.

Ebenso umfasst das erfindungsgemäße System auch an den Masterinkubator oder ein anderes Slavegerät anschließbare Inkubatoren. Ein solcher Inkubator umfaßt ein Gehäuse, eine innerhalb des Gehäuses angeordnete Probenkammer zur Aufnahme eines Probenbehältnisses, eine Temperiereinheit und eine die Temperiereinheit steuernde Steuerelektronik, wobei die Probenkammer mittels der Temperiereinheit beheiz- und/oder kühlbar ist und das Gehäuse eine verschließbare Zugangsöffnung zur Beschickung der Probenkammer aufweist, wobei das Gehäuse geeignet ist, mindestens einen weiteren, vertikal auf diesem gestapelten Inkubator zu tragen. Er ist ferner dadurch gekennzeichnet, daß er ein mit seiner Steuerelektronik zusammenwirkendes Bussystem und zwei mit dem Bussystem verbundene Anschlußelemente aufweist, wobei sein Bussystem über jedes der beiden Anschlußelemente jeweils mit einem Bussystem eines weiteren Inkubators verbindbar ist.

Das erfindungsgemäße System sowie die dabei verwendbaren Inkubatoren werden nachfolgend anhand der Zeichnung näher erläutert. Dabei zeigt
- Fig. 1: ein Ausführungsbeispiel eines erfindungsgemäßen Systems,
- Fig. 2: ein erstes Ausführungsbeispiel eines Inkubators mit ausgefahrener Schublade in perspektivischer Ansicht,
- Fig. 3: eine perspektivische Ansicht von Einzelkomponenten des Ausführungsbeispiels nach Fig. 2,
- Fig. 4: eine Schnittdarstellung durch das Ausführungsbeispiel nach Fig. 2 und 3 bei geschlossener Schublade,
- Fig. 5: eine Explosionszeichnung des Ausführungsbeispiels nach Fig. 2, 3 und 4,
- Fig. 6: eine perspektivische Detailansicht eines zweiten Ausführungsbeispiels eines erfindungsgemäßen Inkubators und
- Fig. 7: eine Explosionszeichnung eines dritten Ausführungsbeispiel eines erfindungsgemäßen Inkubators.

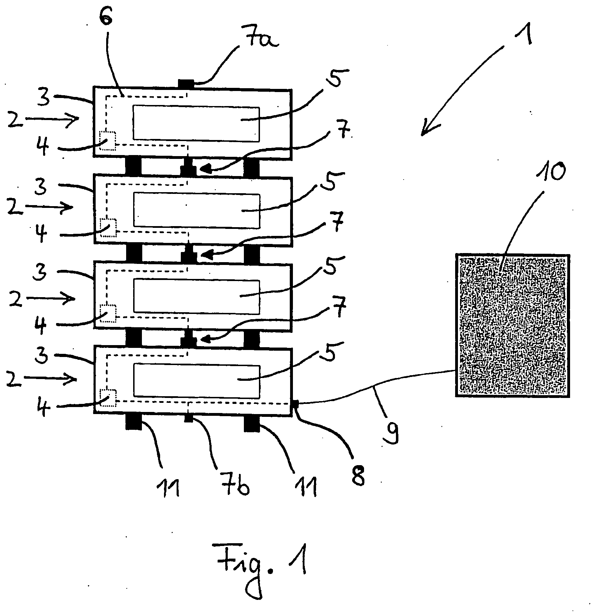

Fig. 1 zeigt ein Ausführungsbeispiel eines erfindungsgemäßen Systems 1 in schematischer Darstellung in Frontansicht. Das System umfaßt mehrere vertikal übereinander gestapelte Inkubatoren 2, die jeweils ein Gehäuse 3 sowie - in Fig. 1 nicht dargestellt - eine mittels einer Temperiereinheit beiheiz- und/oder kühlbare Probenkammer zur Aufnahme eines Probenbehältnisses aufweisen. Die Gehäuse 3 der Inkubatoren umfassen jeweils frontseitig eine - mittels einer Klappe 5 - verschließbare Zugangsöffnung zur Probenkammer. Die jeweilige Temperiereinheit der Inkubatoren 2 ist über eine in den jeweiligen Inkubator 2 integrierte - gestrichelt dargestellte - Steuerelektronik 4 steuerbar. Jeder Inkubator 2 weist außerdem ein mit seiner Steuerelektronik 4 zusammenwirkendes Bussystem 6 (ebenfalls gestrichelt dargestellt) auf. Die in die Inkubatoren 2 integrierten Bussysteme 6 sind über entsprechende Anschlußelemente 7 untereinander verbunden und bilden eine Bussystemarchitektur, die über ein am untersten Inkubator des Stapels angeordnetes Anschlußelement 8 und eine Verbindungsleitung 9, die ggfs. auch durch ein drahtlos funktionierendes Kommunikationsnetz ersetzt werden könnte, mit einer zentralen Steuereinheit 10 verbunden ist. Die zentrale Steuereinheit 10 könnte jedoch alternativ auch an dem Anschlußelement 7b des untersten Inkubators angeschlossen werden. Der unterste Inkubator des Stapels dient vorliegend als Masterinkubator. Über die zentrale Steuereinheit 10 ist ein individuelles Ansteuern der Steuerelektronik 4 sämtlicher Inkubatoren 2 möglich. Die Anschlußelemente 7 liegen vor in Form von jeweils auf der Oberseite der Inkubatoren 2 angeordneten Steckelemente 7a, die mit korrespondierenden Steckelementen 7b auf der Unterseite der Inkubatoren 2 beim Übereinanderstapeln verbunden werden. Jeder der Inkubatoren 2 weist zudem Füße 11 auf, die in hierzu korrespondierende Aussparungen (in Fig. 1 nicht dargestellt) in der Oberseite des darunter liegenden Inkubators eingreifen. Mit Hilfe dieser Aussparungen wird eine exakte Positionierung der Inkubatoren - sowohl horizontal als auch vertikal - sichergestellt.

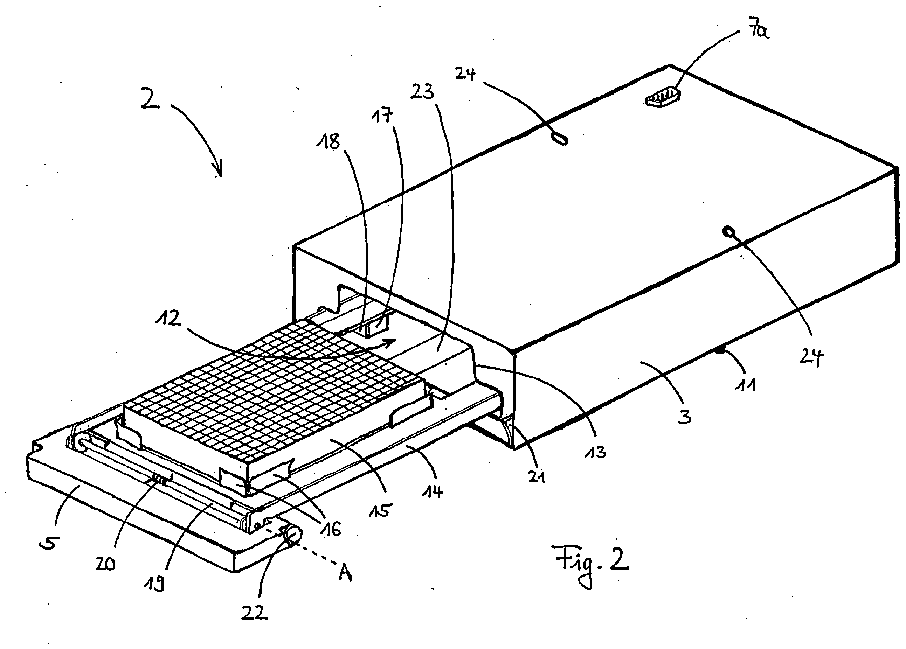

Fig. 2 stellt ein erstes Ausführungsbeispiel eines Inkubators in perspektivischer Ansicht dar. Der Inkubator 2 besitzt ein Gehäuse 3, in dem sich eine Probenkammer 12 zur Aufnahme von Proben befindet. Zum Be- und Entladen der Probenkammer 12 weist das Gehäuse 3 des Inkubators 2 frontseitig eine Zugangsöffnung 13 auf, durch die eine den Boden der Probenkammer 12 bildende Schublade 14 heraus- und hereinfahrbar ist. Die vorliegend vollständig aus der Probenkammer 12 herausgefahrene Schublade 14 trägt ein Probenbehältnis 15, das auf ihr mittels Federklammern 16 gegen ein Verrücken gesichert ist, und schlägt mit einem an der Schublade 14 befestigten Anschlagelement 17 gegen einen am Gehäuse 3 des Inkubators befestigten Anschlag 18 an, wodurch ihre vollständig ausgefahrene Position exakt festlegbar ist.

Die stirnseitig an der Schublade 14 um eine horizontale Achse A verschwenkbar befestigte Klappe 5 befindet sich bei geschlossener Schublade 14 in einer die Zugangsöffnung 13 verschließende, im wesentlichen vertikalen Stellung und ist vorliegend in eine im wesentlichen horizontale Stellung weggeklappt. Dieses Wegklappen erfolgt automatisch beim Herausfahren der Schublade 14 - eingeleitet durch eine am Verbindungselement 19 angebrachte Spiralfeder 20, die sich einerseits gegen die Schublade 14 und andererseits gegen die Klappe 5 abstützt. Das die Achse A bildende Verbindungselement 19 ist an der Klappe nicht randseitig, sondern entfernt von ihrem - in vertikaler Stellung der Klappe gesehenen - unteren und oberen Rand angebracht.

Beim Hereinfahren der Schublade 14 in die Probenkammer 12 wird die Klappe 5 wieder automatisch in eine vertikale Stellung gebracht, in der sie dann die Zugangsöffnung 13 zur Probenkammer 12 verschließt. Dies geschieht mittels einer Kurvenführung 21, an der ein - vorzugsweise drehbar gelagertes - Element 22 der Klappe 5 am Ende des Hereinfahrens der Schublade 14 geführt wird.

Unter dem Probenbehältnis 15 befindet sich eine - in Fig. 2 nicht dargestellte - Heizfolie zum Beheizen der Proben bzw. der Probenkammer 12, die über ein Flachbandkabel 23 mit der - ebenfalls in Fig. 2 nicht dargestellten- Steuerelektronik des Inkubators 2 verbunden ist.

Auf der Oberseite des Gehäuses 3 sind Aufnahmen 24 für Füße 11 eines weiteren Inkubators angeordnet. Um ein Verklemmen der Füße 11 eines weiteren Inkubators in den Aufnahmen 24 zu vermeiden, sind diese derart ausgestaltet, daß eine der Aufnahmen 24 den darin aufgenommenen Fuß 11 - in der horizontalen Ebene - fest lagert, während die andere Aufnahme 24 den in ihr aufgenommenen Fuß in der die beiden Aufnahmen verbindenden Richtung lose lagert. Dies ist in Fig. 2 durch die ovale Ausgestaltung der - links dargestellten - Aufnahme 24 zu erkennen. Ferner ist auf der Oberseite des Inkubators 2 ein Steckelement 7a angeordnet, über das das mit der Steuerelektronik des Inkubators 2 zusammenwirkende Bussystem mit dem Bussystem eines weiteren Inkubators verbindbar ist, wenn dieser auf den Inkubator gestapelt wird.

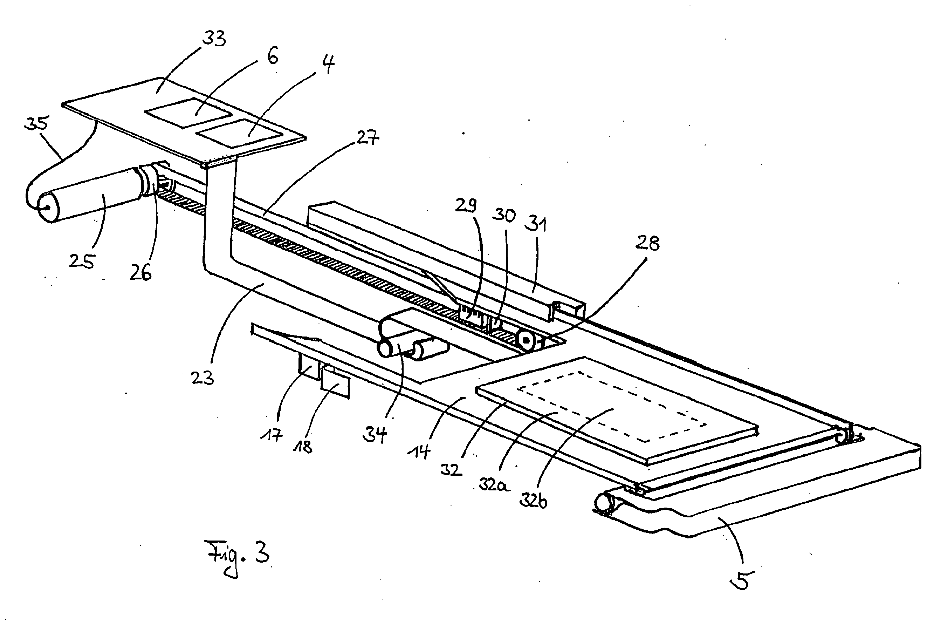

Fig. 3 zeigt ein Ausführungsbeispiel eines Schubladensystems eines Inkubators in perspektivischer Ansicht. Dabei sind zahlreiche Elemente des Inkubators, wie insbesondere dessen Gehäuse, aus Gründen der Übersichtlichkeit nicht dargestellt. Die Schublade 14 wird über einen Elektromotor 25 und einen Zahnriemenantrieb 26, 27, 28 angetrieben. Dabei wird der Zahnriemen 27 über zwei Umlenkrollen 26, 28 geführt, von denen die erste durch den fest am Gehäuse montierten Elektromotor 25 angetrieben ist. Der Zahnriemen 27 ist an der Schublade 14 mittels eines daran befestigten Klemmblocks 29 verklemmt, wobei der Klemmblock 29 zugleich - neben dem ersten Anschlagelement 17 - als zweites Anschlagelement der Schublade 14 dient. Beide Anschlagelemente 17, 29 liegen bei vollständig ausgefahrener Schublade 14 jeweils an einem am Gehäuse befestigten Anschlag 18, 30 des Inkubators an. Die Schublade 14 wird beidseits in Linearführungen 31 geführt, von denen eine der Übersichtlichkeit halber in Fig. 3 nicht dargestellt ist.

Auf der Schublade ist eine Heizfolie 32 zum Beheizen der Probenkammer angeordnet, die über ein Flachbandkabel 23 mit einer Platine 33 verbunden ist. Die Heizfolie 32 ist als Zwei-Zonen-Heizfolie ausgebildet, von der eine erste Zone 32a den Außenbereich und eine zweite Zone 32b den Innenbereich der Heizfolie 32 beheizt. Das Flachbandkabel 23 ist ausgehend von der Heizfolie 32 zunächst von oben durch eine - nicht dargestellte - Aussparung in der ansonsten im wesentlichen vollflächig ausgestalteten Schublade 14 hindurch geführt und im Bereich einer Klemmrolle 34 derart zweifach umgelenkt und durch die Klemmrolle 34 am Boden des Gehäuses befestigt, daß es die Bewegung der Schublade 14 nicht störend beeinflußt.

Auf der Platine 33 sind - schematisch dargestellt - die die Heizfolie 32 ansteuernde Steuerelektronik 4 und das Bussystem 6 des Inkubators angeordnet. Über das Verbindungskabel 35 kann auch der Elektromotor 25 von der Steuerelektronik 4 gesteuert werden. Letztere ist ferner mit dem Bussystem 6 verbunden, das sämtliche der vorbeschriebenen Funktionsmerkmale aufweist und mit zwei Anschlußelementen (in Fig. 3 nicht dargestellt) des Inkubators 2 verbunden ist. Über das Bussystem 6 kann die Steuerelektronik.4 von einer - über eines der Anschlußelemente direkt oder indirekt angeschlossenen - zentralen Steuereinheit angesteuert werden.

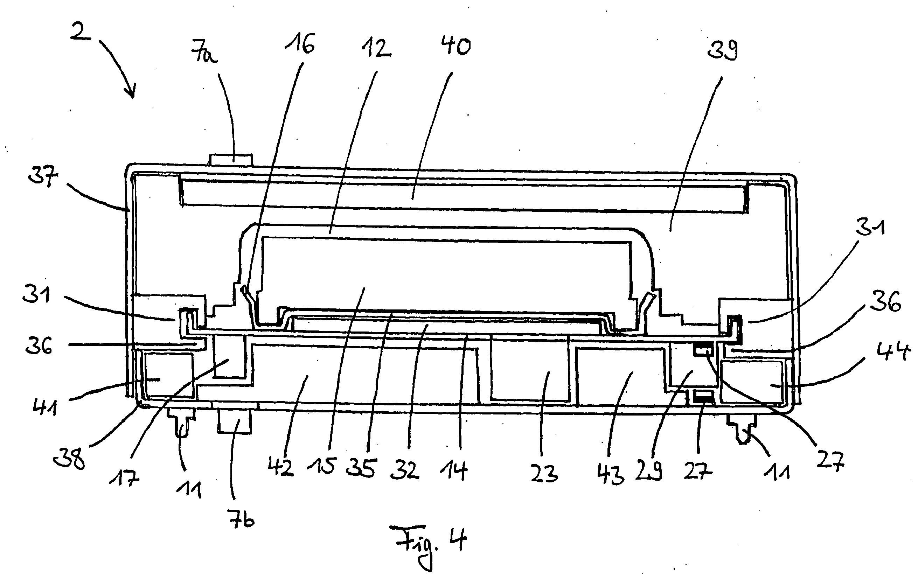

Fig. 4 zeigt eine Schnittdarstellung eines Ausführungsbeispiels eines erfindungsgemäßen Inkubators 2 in einer Schnittebene quer durch die Probenkammer 12. Innerhalb der Probenkammer 12 befindet sich ein Probenbehältnis 15, in welchem sich eine oder mehrere - nicht dargestellte - Proben befinden. Das Probenbehältnis 15 ist in einer auf der Schublade 14 befestigten Aufnahme 35 aufgenommen, die an ihren seitlichen Rändern Federklammern 16 bildet, die das Probenbehältnis 15 in der Aufnahme 35 seitlich fixieren. Zwischen der Schublade 14 und der Aufnahme 35 ist eine Heizfolie 32 zum Beheizen der Probenkammer 12 angeordnet, die über ein Flachbandkabel 23 mit der Steuerelektronik des Inkubators 2 verbunden ist. Durch eine im wesentlichen vollflächige Anlage der Heizfolie 32 an der Aufnahme 35, die wiederum der Unterseite des Probenbehältnisses 15 anliegt, ist ein guter Wärmeübertrag von der Heizfolie auf das Probenbehältnis - und damit auf die sich darin befindliche Probe - sichergestellt. Die Schublade 14 ist beidseits in - am Gehäuse befestigten - Linearführungen 31 geführt, die in vertikaler Richtung beide für eine feste Lagerung der Schublade 14 sorgen. In horizontaler Richtung und quer zur Bewegungsrichtung der Schublade 14 ist die Schublade 14 in der rechts dargestellten Linearführung 31 fest und der links dargestellten Linearführung 31 lose gelagert, um eine gewisse Wärmedehnung der Schublade 14 in dieser Richtung zu erlauben. Die Schublade 14 liegt in ihrem Randbereich einer Auflage 36, die zugleich einen Teil der Linearführung 31 bildet, horizontal verschiebbar auf. Im Interesse einer möglichst guten Gleiteigenschaft der Schublade 14 erweist sich eine Kombination aus Edelstahl für die Schublade 14 und Kunststoff, vorzugsweise PETP-TX mit Teflonanteil, für die Linearführung 31 bzw. die Auflage 36 als besonders günstig. Ebenfalls in Fig. 4 zu erkennen sind die beiden an der Schublade 14 fest angeordneten Anschlagselemente 17, 29, von denen das in der Darstellung rechte Anschlagselement 29 gleichzeitig als Klemmblock zur Verbindung der Schublade 14 mit dem Zahnriemen 27 der Antriebseinheit der Schublade dient. Das Gehäuse umfaßt ein oberes Gehäuseteil 37 und ein unteres Gehäuseteil 38 und weist an seiner Ober- bzw. Unterseite Steckelemente 7a, 7b zur Verbindung seines Bussystems mit dem Bussystem eines darüber- bzw. darunterliegenden Inkubators oder einer zentralen Steuereinheit auf. Zu Isolationszwecken ist die Probenkammer 12 nach oben hin in einer Sandwichstruktur zunächst mit einer ersten Isolationsschicht 39 aus massivem Kunststoff und mit einer zweiten vakuumisolierten Schicht 40 ausgestattet. Die Isolationsschicht 39 aus massivem Kunststoff isoliert die Probenkammer 12 auch zur Seite hin, wobei im-Bereich der Linearführung 31 mit der Auflage 36 für die Schublade 14 eine etwas eingeschränkte Wärmedämmung in Kauf genommen wird. Weitere Elemente 41, 42, 43, 44 aus massivem Kunststoff isolieren die Probenkammer nach unten hin. Auch hier kann ggfs. noch eine zweite vakuumisolierte Schicht unterhalb der Elemente 41 - 44 vorgesehen werden.

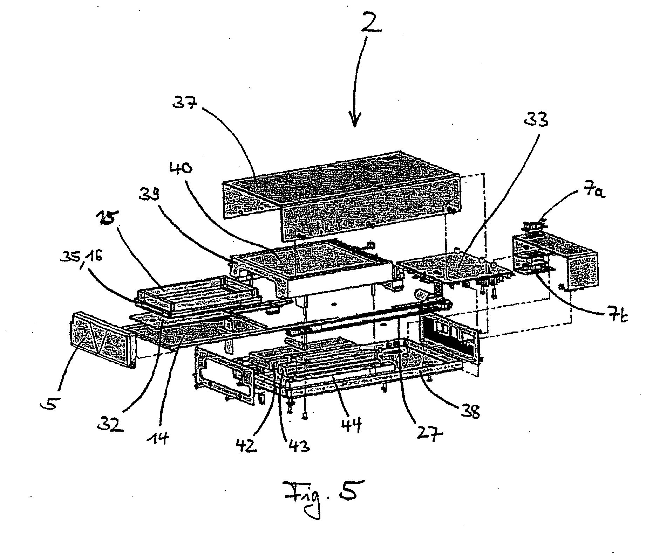

Fig. 5 zeigt zur besseren Übersicht eine Explosionsdarstellung des ersten Ausführungsbeispiels der erfindungsgemäß zur Verwendung kommenden Inkubatoren.

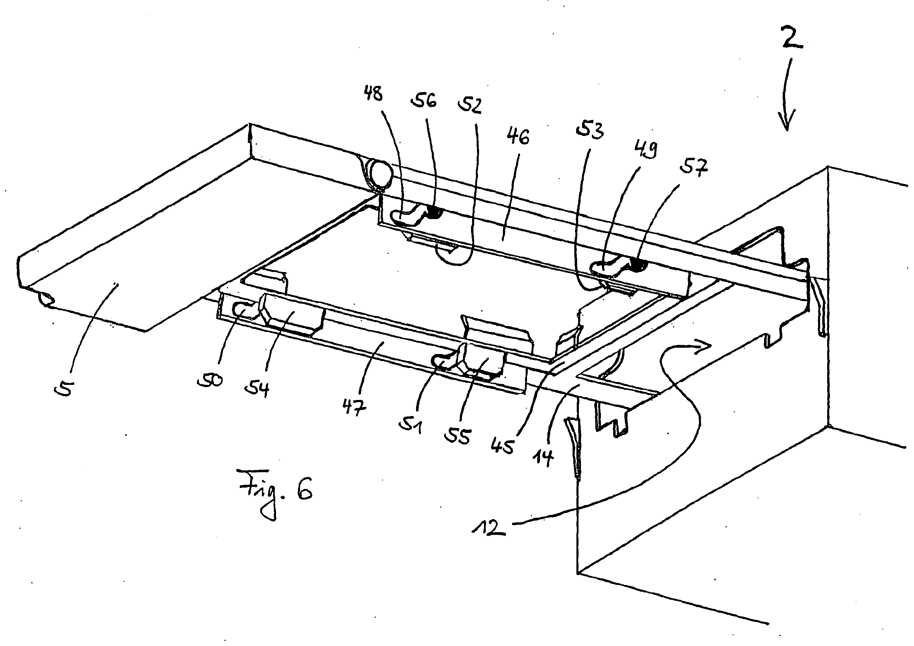

Fig. 6 zeigt eine perspektivische Darstellung eines zweiten Ausführungsbeispiels eines Inkubators 2 von schräg rechts unten, bei dem die Schublade 14 nicht vollflächig, sondern in Form eines Rahmens ausgebildet ist. Dieser Rahmen trägt an seinem inneren Umfang eine Aufnahme 45 für ein Probenbehältnis. An der Schublade 14 ist ferner eine Kulissenführung befestigt, die beidseits jeweils eine Grundplatte 46 bzw. 47 mit darin ausgebildeten Führungen 48, 49 bzw. 50, 51 umfaßt. Die Aufnahme 45 weist vier Führungszapfenhalterungen 52, 53, 54, 55 auf, an denen jeweils ein in Richtung der Kulissenführung weisender Führungszapfen 56, 57 ausgebildet ist. Aus perspektivischen Gründen sind die beiden Führungszapfen 56, 57 zu sehen, die in die Führungen 48, 49 der Kulissenführung eingreifen. Innerhalb des Inkubators 2 ist ferner ein - nicht dargestellter - Anschlag für die Aufnahme 45 bzw. das in der Aufnahme 45 angeordnete und fixierte Probenbehältnis vorgesehen. Dieser Anschlag ist derart angeordnet, daß die Aufnahme 45 bzw. das Probenbehältnis an diesem bereits anschlägt, bevor der Schließvorgang der Schublade 14 abgeschlossen ist. Dadurch werden die Aufnahme 45 und das darin angeordnete Probenbehältnis während des letzten Abschnitts des Schließvorgangs der Schublade 14 relativ zu dieser horizontal verschoben und die Kulissenführung sorgt dafür, daß die horizontal zur Schublade 14 bewegte Aufnahme 45 durch die in den Führungen 48 - 51 bewegten Führungszapfen 56, 57 vertikal abgesenkt wird. Damit kann das Probenbehältnis während des Schließvorgangs der Schublade 14 auf dem Boden der Probenkammer 12 bzw. einer darin angeordneten Zwischenplatte vertikal abgesetzt werden.

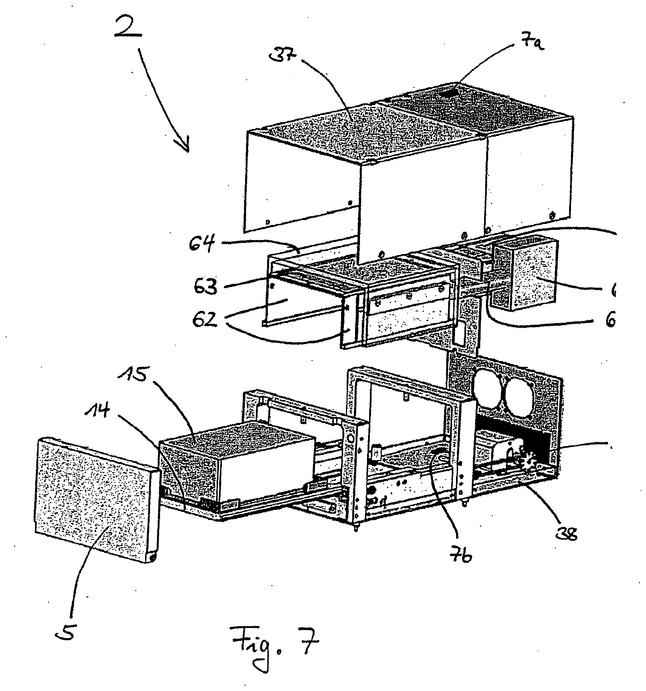

Fig. 7 zeigt schließlich noch eine Explosionsdarstellung eines weiteren Ausführungsbeispiels eines Inkubators 2, dessen Gehäuse ein oberes Gehäuseteil 37 und ein unteres Gehäuseteil 38 umfaßt. Die in einem Probenbehältnis 15 befindliche Probe ist mittels einer durch einen Zahnriemenantrieb 58 angetriebenen Schublade 14 in das Gehäuse des Inkubators 2 herein- und aus diesem wieder herausfahrbar, wobei die hierzu erforderliche frontseitige Zugangsöffnung durch eine an der Frontseite des Gehäuses angeordnete Klappe 5 verschließbar ist. Die Probenkammer ist über ein kombiniertes Kühl-/Heizaggregat 59 in einem Temperaturbereich zwischen +4°C und +80°C (bei Raumtemperatur im Labor) sowohl kühlbar als auch beheizbar. Das kombinierte Kühl-/Heizaggregat ist über jeweils einen Kühl-/Heizflansch 60 und über Heatpipes 61 mit den Seitenwänden 62 der Probenkammer thermisch leitend verbunden. Die Decke 63 der Probenkammer wird nicht direkt gekühlt. Die Probenkammer ist nach oben und zur Seite mit einer - nicht detailliert und transparent dargestellten - Sandwichstruktur 64 isoliert, die von innen nach außen zunächst massiven Kunststoff, dann geschlossen-zelligen Kautschuk und schließlich eine Vakuumisolation aufweist. Die - nicht dargestellte - Steuerelektronik des Inkubators ist über ein Bussystem mit Steckelementen 7a, 7b verbunden, die dem Anschluß des Bussystems des Inkubators 2 an Bussysteme weiterer Inkubatoren dienen.

## Patentansprüche

1. System (1) aus mehreren Inkubatoren (2), die jeweils ein Gehäuse (3), eine innerhalb des Gehäuses (3) angeordnete Probenkammer (12) zur Aufnahme eines Probenbehältnisses (15), eine Temperiereinheit und eine die Temperiereinheit steuernde Steuerelektronik (4) umfassen, wobei die Probenkammern (12) der Inkubatoren (2) mittels der Temperiereinheiten beheiz- und/oder kühlbar sind und die Gehäuse (3) der Inkubatoren (2) jeweils eine verschließbare Zugangsöffnung (13) zum Be- und Entladen der Probenkammer (12) aufweisen, wobei die Gehäuse (3) der Inkubatoren (2) ein vertikales Stapeln der Inkubatoren (2) gestatten, **dadurch gekennzeichnet,**
**daß** die Inkubatoren (2) jeweils ein mit ihrer Steuerelektronik (4) zusammenwirkendes Bussystem (6) aufweisen, wobei die Bussysteme (6) der Inkubatoren (2) über entsprechende Anschlußelemente (7) untereinander verbunden sind, und das System (1) eine zentrale Steuereinheit (10) umfaßt, die mit dem Bussystem (6) eines der Inkubatoren (2) des Systems (1) (Masterinkubator) verbunden ist, wobei über die Bussystemarchitektur ein individuelles Ansteuern der Steuerelektronik (4) sämtlicher Inkubatoren (2) des Systems (1) durch die zentrale Steuereinheit (10) möglich ist, so daß Proben in verschiedenen Probenkammern (12) gleichzeitig einer unterschiedlichen Temperatur ausgesetzt werden können und
**daß** wenigstens ein Inkubator (2) des Systems (1) sich von den anderen Inkubatoren (2) des Systems hinsichtlich seiner Bauart und Funktionalität unterscheidet, indem er neben einer Temperiereinheit wenigstens ein weiteres Mittel zur Beeinflussung weiterer Umgebungsbedingungen für die in der Probenkammer befindlichen Proben aufweist.

2. System aus mehreren Inkubatoren nach Anspruch 1,
**dadurch gekennzeichnet,**
**daß** jeweils zwei benachbarte Inkubatoren (2) untereinander über zwei Anschlußelemente in Form von zueinander korrespondierenden Steckelementen (7a, 7b) verbindbar sind, wobei zumindest bei einem Teil der Inkubatoren (2) die Steckelemente (7a, 7b) derart einander gegenüberliegend an der Oberseite und der Unterseite des Gehäuses (3) angeordnet sind, daß sie mit wiederum korrespondierenden Steckelementen (7a, 7b) des darunter liegenden bzw. darüber gestapelten Inkubators (2) eine Steckverbindung bilden.

3. System aus mehreren Inkubatoren nach Anspruch 1 oder Anspruch 2,
**dadurch gekennzeichnet,**
**daß** zumindest ein Teil der Inkubatoren (2) eine in Linearführungen (31) geführte Schublade (14) zur Be- und Entladung der Probenkammer (12) aufweisen, die mittels eines Motors (25) durch die Zugangsöffnung (13) aus dem Gehäuse (3) des Inkubators (2) heraus- und in diesen hereinfahrbar ist.

4. System aus mehreren Inkubatoren nach Anspruch 3,
**dadurch gekennzeichnet,**
**daß** die Schublade (14) in ihrer ausgefahrenen Endposition an mindestens einem Anschlag (18,30) des betreffenden Inkubators (2) anschlägt.

5. System aus mehreren Inkubatoren nach Anspruch 4,
**dadurch gekennzeichnet,**
**daß** die Zugangsöffnung (13) im Gehäuse (3) der Inkubatoren (2) durch eine stirnseitig an der Schublade (14) befestigte Klappe (5) verschließbare ist.

6. System aus mehreren Inkubatoren nach Anspruch 5,
**dadurch gekennzeichnet,**
**daß** die Klappe (5) bei hereingefahrener Schublade (14) eine im wesentlichen vertikale, die Zugangsöffnung (13) verschließende Stellung einnimmt und entfernt von ihrem in vertikaler Stellung gesehenen unteren oder oberen Rand an der Schublade (14) um eine horizontale Achse (A) derart schwenkbar befestigt ist, daß sie bei herausgefahrener Schublade (14) in eine im wesentlichen horizontale Stellung wegklappt.

7. System aus mehreren Inkubatoren nach einem der Ansprüche 1 bis 6,
**dadurch gekennzeichnet,**
**daß** die Temperiereinheit mindestens eines der Inkubatoren des Systems eine am Boden der Probenkammer (12) angeordnete Heizfolie (32) umfaßt.

8. System aus mehreren Inkubatoren nach Anspruch 7,
**dadurch gekennzeichnet,**
**daß** es sich bei der Heizfolie (32) um eine Zwei-Zonen-Heizfolie (32) handelt oder die Temperiereinheit mindestens zwei Heizfolien umfaßt, mit welchen mindestens zwei Zonen (32a, 32b) jeweils separat beheizbar sind.

9. System aus mehreren Inkubatoren nach einem,der Ansprüche 1 bis 6,
**dadurch gekennzeichnet,**
**daß** die Temperiereinheit mindestens eines der Inkubatoren (2) ein Kühlaggregat (59) zum Kühlen der Probenkammer (12) umfaßt.

10. System aus mehreren Inkubatoren nach einem der Ansprüche 1 bis 6,
**dadurch gekennzeichnet,**
**daß** die Temperiereinheit mindestens eines der Inkubatoren (2) ein kombiniertes Kühl-/Heizaggregat (59) zum Kühlen und Heizen der Probenkammer (12) umfaßt.

11. System aus mehreren Inkubatoren nach Anspruch 9 oder 10,
**dadurch gekennzeichnet,**
**daß** nur der Boden und/oder die Seitenwände (62) der Probenkammer (12) direkt kühlbar sind.

12. System aus mehreren Inkubatoren nach Anspruch 11,
**dadurch gekennzeichnet,**
**daß** der Boden und/oder die Seitenwände (62) der Probenkammer von dem Kühlaggregat oder dem kombinierten Kühl-/Heizaggregat (59) räumlich getrennt und mit diesem über Heatpipes (61) verbunden sind.

13. System aus mehreren Inkubatoren nach einem der Ansprüche 1 bis 12,
**dadurch gekennzeichnet,**
**daß** die Probenkammer (12) mindestens eines der Inkubatoren (2) eine Isolation aufweist, die in einer Sandwichstrukturvon innen nach außen mindestens zwei verschiedene Isolationstypen (38,40) aufweist, von denen die äußere als Vakuum-Isolation (40) ausgeführt ist.

14. System aus mehreren Inkubatoren nach Anspruch 13,
**dadurch gekennzeichnet,**
**daß** die Probenkammer des mindestens einen Inkubators von innen nach außen durch eine Sandwichstruktur (64) aus massivem Kunststoff, geschlossen-zelligem Kautschuk und einer Vakuum-Isolation isoliert ist.

15. System aus mehreren Inkubatoren nach Anspruch 3,
**dadurch gekennzeichnet,**
**daß** die Schublade (14) mindestens eines der Inkubatoren (2) eine mittels einer Kulissenführung (46 - 51, 56, 57) geführte Aufnahme (45) für ein Probenbehältnis (15) umfaßt und die Kulissenführung (46 - 51, 56, 57) zum Abstellen des Probenbehältnisses (15) auf einer Zwischenplatte oder dem Boden der Probenkammer (12) eine vertikale Absenkung der Aufnahme (45) während eines letzten Abschnittes des horizontalen Hereinfahrens derSchublade in die Probenkammer (12) bewirkt.

16. System aus mehreren Inkubatoren nach Anspruch 15,
**dadurch gekennzeichnet,**
**daß** die Zwischenplatte in ihrer Geometrie auf ihrer Unterseite einem durch die Temperiereinheit beheizten und/oder gekühlten Boden der Probenkammer (12) angepaßt und auf ihrer Oberseite der Geometrie eines in die Probenkammer (12) einzubringenden Probenbehältnisses (15) angepaßt ist.

17. System aus mehreren Inkubatoren nach Anspruch 15,
**dadurch gekennzeichnet,**
**daß** die Zwischenplatte in der Probenkammer (12) innerhalb der horizontalen Ebene bewegbar angeordnet ist und mittels eines Antriebssystems zu Schwingungen innerhalb der horizontalen Ebene anregbar ist.

## Claims

1. System (1) consisting of multiple incubators (2), which each comprise a housing (3), a sample chamber (12) arranged within the housing (3) for receiving a sample receptacle (15), a tempering unit and a set of drive electronics (4) driving the tempering unit, wherein the sample chambers (12) of the incubators (2) can be heated and/or cooled by means of the tempering units, and the housings (3) of the incubators (2) each have a lockable access opening (13) for loading and unloading the sample chamber (12), the housings (3) of the incubators (2) allowing a vertical stacking of the incubators (2),
**characterized in that**
the incubators (2) each have a bus system (6) interacting with their drive electronics (4), wherein the bus systems (6) of the incubators (2) are interconnected via corresponding connection elements (7), and the system (1) comprises a central drive unit (10) that is connected to the bus system (6) of one of the incubators (2) of the system (1) (master incubator), wherein an individual control of the drive electronics (4) of all incubators (2) of the system (1) is possible using the bus system architecture by means of said drive unit (10), so that samples in different sample chambers (12) can be simultaneously exposed to a different temperature and
that at least one incubator (2) of the system (1) differs from the other incubators (2) of the system in respect of its design and functionality, since in addition to a tempering unit it has a further means for manipulating other environmental conditions for the samples located in the sample chamber.

2. System consisting of multiple incubators according to Claim 1,
**characterized in that**
any two neighbouring incubators (2) can be connected together via two connection elements in the form of plug-in elements corresponding to each other (7a, 7b), wherein at least for some of the incubators (2), the plug-in elements (7a, 7b) are arranged opposite one another on the top and underside of the housing (3) in such a manner that they form a plug and socket connection with likewise corresponding plug-in elements (7a, 7b) of the incubator (2) stacked above or below them, respectively.

3. System consisting of multiple incubators according to Claim 1 or Claim 2,
**characterized in that**
at least some of the incubators (2) have a drawer (14) guided in linear guides (31) for loading and unloading the sample chamber (12), which can be moved out of and inserted into the housing (3) of the incubator (2) through the access opening (13) by means of a motor (25) .

4. System consisting of multiple incubators according to Claim 3,
**characterized in that**
in its fully opened end position, the drawer (14) rests against at least one stop (18, 30) of the relevant incubator (2).

5. System consisting of multiple incubators according to Claim 4, **characterized in that**
the access opening (13) in the housing (3) of the incubators (2) is lockable by means of a flap (5) attached to the front face of the drawer (14).

6. System consisting of multiple incubators according to Claim 5,
**characterized in that**
when the drawer (14) is closed, the flap (5) assumes an essentially vertical position, locking the access opening (13), and is fixed to the drawer (14) away from its lower or upper edge as viewed in the vertical position so that it can be pivoted about a horizontal axis (A), in such a way that when the drawer (14) is fully open, it folds out into an essentially horizontal position.

7. System consisting of multiple incubators according to any one of Claims 1 to 6,
**characterized in that**
the tempering unit of at least one of the incubators of the system comprises a heating foil (32) arranged on the floor of the sample chamber (12).

8. System consisting of multiple incubators according to Claim 7,
**characterized in that**
the heating foil (32) is a two-zone heating foil (32), or the tempering unit comprises at least two heating foils, with which at least two zones (32a, 32b) can be respectively separately heated.

9. System consisting of multiple incubators according to any one of Claims 1 to 6,
**characterized in that**
the tempering unit of at least one of the incubators (2) comprises a cooling assembly (59) for cooling the sample chamber (12).

10. System consisting of multiple incubators according to any one of Claims 1 to 6,
**characterized in that**
the tempering unit of at least one of the incubators (2) comprises a combined cooling/heating assembly (59) for cooling and heating the sample chamber (12).

11. System consisting of multiple incubators according to Claim 9 or 10,
**characterized in that**
the floor and/or the side walls (62) of the sample chamber (12) exclusively can be directly cooled.

12. System consisting of multiple incubators according to Claim 11,
**characterized in that**
the floor and/or the side walls (62) of the sample chamber are spatially separated from the cooling assembly or the combined cooling/heating assembly (59) and connected thereto via heat pipes (61).

13. System consisting of multiple incubators according to any one of Claims 1 to 12,
**characterized in that**
the sample chamber (12) of at least one of the incubators (2) has insulation which has at least two different insulation types (38, 40) in a sandwich structure from inside to outside, the external one of which is implemented as a vacuum insulation (40).

14. System consisting of multiple incubators according to Claim 13, **characterized in that**
the sample chamber of the at least one incubator is insulated from inside to outside by a sandwich structure (64) made of solid plastic, closed-cellular rubber and a vacuum insulation.

15. System consisting of multiple incubators according to Claim 3,
**characterized in that**
the drawer (14) of at least one of the incubators (2) comprises a holder (45), guided by means of a sliding guide (46 - 51, 56, 57), for a sample receptacle (15), and to deposit the sample receptacle (15) on an intermediate plate or the floor of the sample chamber (12) the sliding guide (46 - 51, 56, 57) causes a vertical lowering of the holder (45) during a final section of the horizontal insertion of the drawer into the sample chamber (12).

16. System consisting of multiple incubators according to Claim 15,
**characterized in that**
the underside of the intermediate plate is adapted in its geometry to a floor of the sample chamber (12) that is heated and/or cooled by the tempering unit, and on its top is adapted to the geometry of a sample receptacle (15) to be inserted into the sample chamber (12) .

17. System consisting of multiple incubators according to Claim 15,
**characterized in that**
the intermediate plate in the sample chamber (12) is movable within the horizontal plane and can be excited to oscillation within the horizontal plane by means of a drive system.

## Revendications

1. Système (1) constitué de plusieurs incubateurs (2), comprenant chacun un boîtier (3), une chambre d'essai (12) disposée à l'intérieur du boîtier (3) pour l'admission d'un récipient à échantillons (15), une unité de régulation de température et une électronique de commande (4) pour commander l'unité de régulation de température, les chambres d'essai (12) des incubateurs (2) pouvant être réchauffées et/ou refroidies, et les boîtiers (3) des incubateurs (2) comportant chacun une ouverture d'accès refermable (13), pour le chargement et le déchargement de la chambre d'essai (12), les boîtiers (3) des incubateurs (2) permettant d'empiler verticalement les incubateurs (2),
**caractérisé en ce que**
les incubateurs (2) comportent chacun un système de bus (6) coopérant avec son électronique de commande (4), les systèmes de bus (6) des incubateurs (2) étant reliés entre eux par des éléments de raccordement (7) correspondants, et le système (1) comprenant une unité de commande centrale (10), qui est reliée avec le système de bus (6) de l'un des incubateurs (2) du système (1) (incubateur maître), l'architecture du système de bus permettant un actionnement individuel de l'électronique de commande (4) de l'ensemble des incubateurs (2) du système (1), par le biais de l'unité de commande centrale (10), de sorte que des échantillons peuvent être soumis chacun à une température différente, simultanément, dans plusieurs chambres d'essai (12), et
**en ce qu'**au moins un incubateur (2) du système (1) se distingue des autres incubateurs (2) du système de par sa conception et sa fonctionnalité, du fait qu'en plus d'une unité de régulation de température, il présente un moyen supplémentaire destiné à influencer d'autres conditions ambiantes pour les échantillons situés dans la chambre d'essai.

2. Système constitué de plusieurs incubateurs selon la revendication 1,
**caractérisé en ce que**
deux incubateurs (2) voisins peuvent être reliés entre eux par deux éléments de raccordement sous la forme d'éléments d'emboîtement (7a, 7b) correspondant les uns avec les autres, les éléments d'emboîtement (7a, 7b) d'au moins une partie des incubateurs (2) étant disposés de telle manière l'un en face de l'autre sur le dessus et le dessous du boîtier (3), qu'ils forment une liaison par emboîtement avec des éléments d'emboîtement (7a, 7b), également correspondants, de l'incubateur (2) empilé en-dessous ou au-dessus.

3. Système constitué de plusieurs incubateurs selon l'une des revendications 1 ou 2,
**caractérisé en ce que**
au moins une partie des incubateurs (2) comportent un tiroir (14) guidé dans des guidages linéaires (31), pour le chargement et le déchargement de la chambre d'essai (12), qui peut être introduit dans et sorti du boîtier (3) de l'incubateur (2), par l'ouverture d'accès (13), au moyen d'un moteur (25).

4. Système constitué de plusieurs incubateurs selon la revendication 3,
**caractérisé en ce que**
le tiroir (14) bute contre au moins une butée (18, 30) de l'incubateur (2) concerné, dans sa position sortie terminale.

5. Système constitué de plusieurs incubateurs selon la revendication 4,
**caractérisé en ce que**
l'ouverture d'accès (13) dans le boîtier (3) des incubateurs (2) peut être fermée par un clapet (5) fixé frontalement au tiroir (14).

6. Système constitué de plusieurs incubateurs selon la revendication 5,
**caractérisé en ce que**
lorsque le tiroir (14) est rentré, le clapet (5) prend une position essentiellement verticale fermant l'ouverture d'accès (13), tout en étant fixé au tiroir (14), de façon pivotante autour d'un axe horizontal (A), loin de son bord inférieur ou supérieur du point de vue de sa position verticale, de manière à être rabattu, dans une position essentiellement horizontale, lorsque le tiroir (14) est sorti.

7. Système constitué de plusieurs incubateurs selon l'une des revendications 1 à 6,
**caractérisé en ce que**
l'unité de régulation de température d'au moins l'un des incubateurs du système comprend un film chauffant (32) disposé au fond de la chambre d'essai (12).

8. Système constitué de plusieurs incubateurs selon la revendication 7,
**caractérisé en ce que**
le film chauffant (32) est un film chauffant à deux zones (32) ou **en ce que** l'unité de régulation de température comprend au moins deux films chauffants, avec lesquels au moins deux zones (32a, 32b) peuvent être chauffées séparément.

9. Système constitué de plusieurs incubateurs selon l'une des revendications 1 à 6,
**caractérisé en ce que**
l'unité de régulation de température d'au moins l'un des incubateurs (2) comprend un agrégat de refroidissement (59) pour le refroidissement de la chambre d'essai (12).

10. Système constitué de plusieurs incubateurs selon l'une des revendications 1 à 6,
**caractérisé en ce que**
l'unité de régulation de température d'au moins l'un des incubateurs (2) comprend un agrégat de chauffage/refroidissement (59) pour le refroidissement et le chauffage de la chambre d'essai (12).

11. système constitué de plusieurs incubateurs selon l'une des revendications 9 ou 10,
**caractérisé en ce que**
seul (s) le fond et/ou les parois latérales (62) de la chambre d'essai peuvent être refroidis directement.

12. Système constitué de plusieurs incubateurs selon la revendication 11,
**caractérisé en ce que**
le fond et/ou les parois latérales (62) de la chambre d'essai sont spatialement séparées de l'agrégat de refroidissement ou de l'agrégat combiné de refroidissement/chauffage (59), et relié(es) à celui-ci par des Heat Pipes (61).

13. Système constitué de plusieurs incubateurs selon l'une des revendications 1 à 12,
**caractérisé en ce que**
la chambre d'essai d'au moins l'un des incubateurs (2) est isolée depuis l'intérieur par rapport à l'extérieur par une structure de sandwich (64) constituée d'un plastique dur, d'un caoutchouc à pores fermés et d'une isolation par le vide.

14. Système constitué de plusieurs incubateurs selon la revendication 13,
**caractérisé en ce que**
la chambre d'essai de l'au moins un incubateur est isolée de l'intérieur par rapport à l'extérieur, par une structure de sandwich (64) constituée d'une matière plastique massive, d'un caoutchouc à pores fermés et d'une isolation par le vide.

15. Système constitué de plusieurs incubateurs selon la revendication 3,
**caractérisé en ce que**
le tiroir (14) d'au moins l'un des incubateurs (2) comprend une admission (45), guidé au moyen d'un guidage à coulisse (46-51, 56, 57) et destiné à un récipient à échantillons (15), et le guidage à coulisse (46-51, 56, 57) produit un abaissement vertical de l'admission (45), sur une dernière partie de l'introduction horizontale du tiroir dans la chambre d'essai (12), pour le dépôt du récipient à échantillon (15) sur une plaque intermédiaire ou sur le fond de la chambre d'essai (45).

16. Système constitué de plusieurs incubateurs selon la revendication 15,
**caractérisé en ce que**
de par sa géométrie, la face inférieure de la plaque intermédiaire est adaptée à un fond de la chambre d'essai (12), qui est chauffé et/ou refroidi par l'unité de régulation de température, et la face supérieure est adaptée à la géométrie d'un récipient à échantillons (15) à introduire dans la chambre à échantillons (12).

17. Système constitué de plusieurs incubateurs selon la revendication 15,
**caractérisé en ce que**
la plaque intermédiaire est disposée de façon mobile sur le plan horizontal dans la chambre d'essai (12), et peut être activée au moyen d'un système d'entraînement, de façon à osciller sur le plan horizontal.
